# EUROPEAN PATENT SPECIFICATION

(11) **EP 2 396 292 B1**
(45) Date of publication and mention of the grant of the patent: **13.12.2017**
(21) Application number: 10706745.6
(22) Date of filing: 10.02.2010
(51) Int. Cl.: C07C 2/66, C07C 15/073, C07C 15/085

(54) **INTEGRATED PROCESS FOR THE PREPARATION OF ETHYLBENZENE AND CUMENE**
INTEGRIERTES VERFAHREN ZUR HERSTELLUNG VON ETHYLBENZOL UND CUMOL
PROCÉDÉ INTÉGRÉ POUR LA PRÉPARATION D'ÉTHYLBENZÈNE ET DE CUMÈNE

(30) Priority: 16.02.2009 IT MI20090202
(43) Date of publication of application: 21.12.2011
(73) Proprietor: versalis S.p.A., 20097 San Donato Milanese (MI) (IT)
(72) Inventor: BENCINI, Elena, I-46030 Virgilio (Mantova) (IT); DEL SEPPIA, Alessandro, I-46047 Porto Mantovano (Mantova) (IT)
(74) Representative: Bottero, Carlo
(86) International application number: PCT/IB2010/000273
(87) International publication number: WO 2010/092466

(56) References cited:
- EP-A- 0 366 515
- EP-A1- 0 046 678
- WO-A-99/08984

## Description

WO 99/08984 discloses a process of production of cumene and ethylbenzene in which a mixture of ethylene and propene are fed together in the first step of alkylation, the cumene is then separated and the alkylation of benzene with ethylene proceed in a second reaction step.

The present invention relates to an integrated process for preparing cumene and ethylbenzene through alkylation of benzene comprising the following stages:
(a) contacting benzene and ethylene in the presence of a catalyst containing one or more zeolites,
(b) contacting the mixture resulting from the previous stage with propylene, and possibly with benzene, in the presence of a catalyst containing one or more zeolites,
(c) subjecting the mixture resulting from stage (b) to separation in order to isolate at least one fraction containing ethylbenzene and a fraction containing cumene.

In stage (c) a fraction containing polyalkylbenzenes to be sent to a transalkylation step for the recovery of further ethylbenzene and cumene, can also be isolated.

Cumene, or isopropyl benzene, is an important intermediate product of basic chemical industries, mainly used as precursor for the preparation of phenol, in turn useful as an intermediate product in the preparation of caprolactam from which nylon is produced.

The industrial synthesis of phenol comprises the following stages: alkylation of benzene to cumene, oxidation of cumene to cumene hydroperoxide and subsequent re-arrangement to give phenol and acetone.

Ethylbenzene is also an important intermediate product of basic chemical industries, mainly used as precursor for the production of styrene, in turn useful as intermediate in the preparation of styrene polymers and copolymers.

The industrial synthesis of styrene comprises the stages of alkylation of benzene to ethylbenzene and the transformation of ethylbenzene into styrene by a dehydrogenation reaction.

As far as the alkylation of benzene to cumene or ethylbenzene is concerned, catalysts based on phosphoric acid and infusorial earth for fixed bed reactors or AlCl₃ in slurry, are still widely used in the petrochemical industry.

Problems relating to environmental impact and safety, however, are linked to these processes: the use of these catalysts, in fact, is particularly problematic due to corrosion, the by-production of toxic organic products and the disposal of the exhausted catalysts.

In 1965 the preparation of cumene was described for the first time using as alternative catalyst zeolite X or zeolite Y (Minachev, Kr. M., et al, Neftekhimiya 5 (1965) 676). Subsequently, Venuto et al. (J.Catal.5, (1966) 81) described the use of zeolites having a faujasite structure for the alkylation of benzene by means of light olefins such as propylene. US 4,292,457 describes the use of zeolites of the ZSM-5 type for alkylating benzene with propylene.

Optimum industrial results have been obtained in the synthesis of cumene or ethylbenzene using zeolites with beta type structures, as described in EP 432814, in particular using catalysts comprising beta zeolite according to what is described in EP 687500 and EP 847802.

Once cumene has been obtained, the same is transformed to phenol by means of an oxidation stage to cumene hydroperoxide, followed by an acid treatment stage which causes the breakage of the peroxide bond with the formation of phenol and acetone.

Phenol is mainly used in the preparation of Bisphenol A (about 35%), phenolic resins (about 35%), caprolactam (about 15%), aniline, alkylphenols, xylenols etc., whereas acetone is mainly used in the production of methyl methacrylate (about 45%), Bisphenol A (about 20%), solvents (about 17%) and methyl isobutyl ketone (about 8%).

As far as ethylbenzene is concerned, once this has been obtained, it is transformed into styrene by a dehydrogenation reaction and the product obtained is used in the preparation of plastic materials such as crystal or expandable polystyrene homopolymer, SAN copolymers and SBS rubbers, biphasic ABS or HIPS copolymers.

An integrated process has been now found for the production of ethylbenzene and cumene which, with respect to the single alkylation processes of benzene to cumene and alkylation of benzene to ethylbenzene, allows the maximum exploitation of the plant, with advantages in terms of consumption of raw material and energy and a cost reduction, unexpectedly allowing optimum results to be obtained in terms of yield and selectivity.

An object of the present invention therefore relates to an integrated process for preparing cumene and ethylbenzene through alkylation of benzene comprising the following stages:
(a) contacting benzene and ethylene in the presence of a catalyst containing one or more zeolites,
(b) contacting the mixture resulting from the previous stage with propylene, and possibly with benzene, in the presence of a catalyst containing one or more zeolites,
(c) subjecting the mixture resulting from stage (b) to separation in order to isolate at least one fraction containing ethylbenzene and a fraction containing cumene.

With the integrated process of the present invention, optimum results are unexpectedly obtained in terms of yield and selectivity, even though there is no separation or purification stage between the alkylation stages (a) and (b). In particular, the formation of hypothetically favoured by-products such as ethyl isopropyl benzene and heavy unrecoverable by-products such as diphenylethanes, alkyl diphenylethanes, diphenylpropanes and alkyl diphenylpropanes, is unexpectedly limited. As demonstrated hereunder, the optimum results in terms of yield and selectivity can be further increased by the recovery of the polyalkylbenzene by-products by means of a single transalkylation stage. Furthermore, the integrated process of the present invention does not necessarily require the feeding of fresh benzene in the alkylation stage (b), whereas it possibly allows the re-use in this stage of benzene deriving from the separation stage (c); it is therefore evident that the integrated process of the present invention requires a lower quantity of benzene than that necessary in the two alkylation processes of benzene to cumene and benzene to ethylbenzene considered separately.

According to a preferred aspect of the present invention, in order to maximize the formation of cumene and ethylbenzene, the product obtained from stage (b) can be separated into a fraction (1) containing benzene, a fraction (2) containing ethylbenzene, a fraction (3) containing cumene, a fraction (4) containing polyalkylbenzenes. The fraction (1) can be optionally re-fed to stage (a) and/or (b), and the fraction (4) can be optionally re-fed to stage (a) and/or (b), to undergo at least partial transalkylation to give further cumene and ethylbenzene.

In particular, with respect to stage (b) of the process of the present invention, this can be effected without any addition of further benzene, other than that already contained in the mixture resulting from stage (a), or adding further benzene. In this second case, fresh benzene can be used or the fraction (1) containing benzene deriving from stage (c) or both: a preferred aspect is to use fraction (1) alone containing benzene deriving from stage (c).

According to a particularly preferred aspect, the transalkylation of the fraction of polyalkylates deriving from stage (c) is effected in a specific reactor, where this fraction of polyalkylates is put in contact with a feedstock of benzene, in the presence of a transalkylation catalyst containing a zeolite. Also with respect to the transalkylation stage, optimum results are obtained in terms of yield and selectivity, even if the fraction (4) fed to the transalkylation stage contains products deriving from both the polyalkylation of benzene with ethylene and the polyalkylation of benzene with propylene, in addition to a small quantity of mixed polyalkylated compounds containing both ethyl and propyl groups.

In accordance with this, a particularly preferred aspect of the present invention therefore relates to a process comprising the following stages:
(a) contacting benzene and ethylene, in presence of a catalyst containing one or more zeolites,
(b) contacting the mixture resulting from the previous stage with propylene, and possibly with benzene, in presence of a catalyst containing one or more zeolites,
(c) subjecting the mixture resulting from stage (b) to separation in order to isolate at least: (1) a fraction containing benzene, (2) a fraction containing ethylbenzene, (3) a fraction containing cumene, (4) a fraction containing polyalkylbenzenes;
(d) contacting the fraction (4) containing polyalkylbenzenes with benzene, in presence of a catalyst containing a zeolite, under transalkylation conditions, to obtain cumene and ethylbenzene.

Also when the integrated process comprises the transalkylation stage, it is preferable to use ethylene in stage (a) and propylene in stage (b), and therefore the integrated process according to the present invention comprises the following stages:
(a) contacting benzene and ethylene, in presence of a catalyst containing one or more zeolites,
(b) contacting the mixture resulting from the previous stage with propylene, and possibly with benzene, in presence of a catalyst containing one or more zeolites,
(c) subjecting the mixture resulting from stage (b) to separation in order to isolate at least: (1) a fraction containing benzene, (2) a fraction containing ethylbenzene, (3) a fraction containing cumene, (4) a fraction containing polyalkylbenzenes;
(d) contacting the fraction (4) containing polyalkylbenzenes with benzene, in presence of a catalyst containing a zeolite, under transalkylation conditions, to obtain cumene and ethylbenzene.

Also in this case, fresh benzene can be fed to stage (b) and/or the benzene fraction obtained from the separation of stage (c) can be re-fed to stage (a) and/or (b), and/or (d).

The catalysts used in stage (a) and (b) can be the same or different, they can contain one or more zeolites known to experts for the alkylation of aromatic hydrocarbons with olefins. A single zeolite is preferably used. Zeolites which can be well-used are selected from beta zeolite, zeolite Y , MCM-22, zeolite ZSM-12 and mordenite. These zeolites are described in "Atlas of zeolite structure types", Ch. Baerlocher, W.M.Meier and D.H.Olson, 2001, 5th Edition, Elsevier.

The zeolites are used in acid form, i.e. in the form in which all the negative charges deriving from the aluminum present in the structure are counterbalanced by hydrogen ions, or prevalently acid.

A preferred aspect is to use the same zeolite in both stages (a) and (b), and in particular, a preferred aspect is to use beta zeolite in both stage (a) and stage (b).

The beta zeolite which can be conveniently used in the catalyst of the present invention corresponds to that described in US 3,308,069, and is a porous crystalline material having the composition

[(x/n) M (1±0.1-x) TEA] AlO₂.ySiO₂.wH₂O

wherein n is the oxidation state of M, x is lower than 1, y ranges from 5 to 100, w from 0 to 4, M is a metal selected from those of groups IA, IIA, IIIA of the Periodic System or from transition metals and TEA is tetra-ethylammonium hydroxide.

A preferred aspect of the present invention is for the beta zeolite to be in acid form, i.e. in the form in which the H⁺ ion has partially or totally substituted the metallic cations initially present.

This substitution is effected in accordance with known methods by means of an exchange with ammonium ions, washing and subsequent calcination.

The catalysts which can be used in stages (a) and (b) of the integrated process of the present invention can comprise suitable binding agents, for example oxides of groups IIIA, IVA and IVB. More preferably, the catalytic system can contain an oxide of Si or Al as a binding carrier. Even more preferably, the catalytic system can contain γ-alumina as binding carrier. γ-alumina is a known material commercially available in the form, preferred for the purposes of the present invention, of the precursors bohemite or p-bohemite, subsequently transformed into γ-alumina during the preparation of the catalytic system, in the final calcination phase. The ligand is preferably used in a relative weight quantity with respect to the catalytic system ranging from 5:95 to 95:5. A particularly preferred aspect of the present invention is to use the catalytic compositions containing beta zeolite described in EP 687500 and EP 847802: i.e. catalytic compositions containing beta zeolite and an inorganic ligand characterized in that the extra-zeolitic porosity is such as to consist for a fraction of at least 25% of pores with a radius higher than 100 Å (EP 687500), and catalytic compositions containing beta zeolite and an inorganic ligand having an extra-zeolitic porosity is such as to consist for a fraction of at least 25% of pores with a radius higher than 100 Å, and characterized by a total volume of extra-zeolitic pores greater than or equal to 0.80 ml/g.

The alkylation reactions of stage (a) and (b) can be carried out in continuous, semi-continuous or batchwise. The reactions can be effected in gaseous phase, liquid phase or mixed phase. A particularly preferred aspect is to operate in continuous and in liquid phase in both stage (a) and stage (b). In order to maintain the temperature within a preferred range and reduce the by-production of polyalkylates, the catalyst can be arranged in the rector in various layers; a quench is effected between one layer and another, with inert solvents and/or part of the benzene and/or part of the olefin. By suitably operating, high benzene/olefin ratios can be obtained on the single layer, without increasing the same overall ratio, with evident advantages on the subsequent separation and possible recyclings. The alkylation reactions of stages (a) and (b) can be suitably carried out in two or more reactors in series, inter-cooled to control the temperature. The feeding of the olefin and/or aromatic compound can be conveniently distributed between the various reactors and different reactor layers; it can possibly be diluted with benzene or with an inert product to favour the temperature control.

According to a preferred aspect, the reactions of stages (a) and (b) are effected at a reaction temperature ranging from 100 to 300 °C, preferably from 120 to 250 °C. The pressure is generally equal to or higher than atmospheric pressure and preferably ranges from 10 to 50 bar, even more preferably from 20 to 45 atm; the WHSV space velocity ranges from 0.1 to 10 hours⁻¹.

In stage (a), the feeding of the olefin preferably corresponds to a Benzene/olefin molar ratio ranging from 1 to 20, even more preferably from 2 to 8. In stage (b), the feeding of the olefin preferably corresponds to a benzene/olefin molar ratio ranging from 1 to 20, even more preferably ranging from 2 to 8, wherein benzene refers to that contained in the mixture deriving from step (a), and possibly fresh benzene additionally added and/or recycled benzene contained in the fraction (1) deriving from the separation step (c).

In particular, in accordance with the present invention, in stage (b), a mixture containing benzene, ethylbenzene and diethylbenzene is fed when ethylene is used in stage (a), whereas a mixture containing benzene, cumene and diisopropylbenzene is fed when propylene is used in stage (a).

The separation stage (c) can be effected using any of the methods known to experts in the field. The product obtained can be fractionated, for example, in a separation section S using conventional separation methods, such as for example, degassing, distillation and demixing of liquids, to obtain a first fraction mainly containing benzene, a second fraction mainly containing ethylbenzene, a third fraction mainly containing isopropylbenzene and a fourth fraction mainly containing polyalkylbenzenes.

The fraction of polyalkylbenzenes mainly contains diethylbenzene, diisopropylbenzene and ethyl-isopropylbenzene.

The first fraction can be re-used in stages (a) and/or (b), and/or in the transalkylation stage (d), when present, where it is added to the fresh benzene feed to react with the fourth fraction, containing polyalkylbenzenes, and further produce the desired products isopropylbenzene and ethylbenzene.

The transalkylation of stage (d) is effected by putting the fraction (4) containing polyalkylbenzenes in contact with benzene, in the presence of a catalyst containing a zeolite, under transalkylation conditions to obtain cumene and ethylbenzene.

The benzene used in this stage can at least partly derive from the benzene contained in the fraction (1) deriving from the separation stage (c). The reaction is carried out in the presence of a catalyst containing zeolite: any zeolite known to experts in the field as transalkylation catalyst can be used in said stage (d). A zeolite selected from zeolite Y and beta zeolite is preferably used.

A preferred aspect is to use a catalyst containing zeolite Y.

The catalysts which can be used in stage (d) of the integrated process of the present invention can comprise suitable binding agents, for example oxides of groups IIIA, IVA and IVB. More preferably, the catalytic system can contain an oxide of Si or Al as ligand carrier. Even more preferably, the catalytic system can contain γ-alumina as ligand carrier. γ-alumina is a known material, commercially available in the form, preferred for the purposes of the invention, of the precursors bohemite or p-bohemite, subsequently transformed into γ-alumina during the preparation of the catalytic system, in the final calcination phase. The ligand is preferably used in a relative weight quantity with respect to the catalytic system ranging from 5:95 to 95:5. When beta zeolite is used in stage (d), a preferred aspect is to use the catalytic compositions containing beta zeolite described in EP 687500 and EP 847802. When, according to a particularly preferred aspect, zeolite Y is used in stage (d), the catalyst containing zeolite Y used can be that described in US2006/0258893, i.e. a catalytic composition comprising zeolite Y and an inorganic ligand, wherein the inorganic ligand is γ-alumina, characterized by a pore volume, obtained by adding the mesoporosity and macroporosity fractions present in the same catalytic composition, higher than or equal to 0.7 cc/g, wherein at least 30% of said volume consists of pores with a diameter greater than 100 nanometres.

The temperature conditions for the transalkylation reaction can be selected from 100 to 350°C, the pressure is selected from 10 to 50 atm and the WHSV ranges from 0.1 to 200 hours⁻¹. Transalkylation conditions which can be conveniently used are, for example, those described in US2006/0258893, in EP 687500 and in EP 847802.

The transalkylation reaction product is fractionated using conventional separation methods, for example those described above with reference to stage (c). In particular, a preferred aspect is to use the same separation section used for stage (c), feeding the mixture resulting from stage (d), to said stage (c).

In accordance with this, a further preferred aspect of the present invention relates to a process comprising the following stages:
(a) contacting benzene and ethylene, in presence of a catalyst containing one or more zeolites,
(b) contacting the mixture resulting from the previous stage with propylene, and possibly with benzene, in presence of a catalyst containing one or more zeolites,
(c) subjecting the mixtures resulting from stage (b) and from stage (d) to separation in order to isolate at least: (1) one fraction containing benzene, (2) one fraction containing ethylbenzene, (3) one fraction containing cumene, (4) one fraction containing polyalkylbenzenes;
(d) contacting the fraction (4) containing the polyalkylbenzenes with benzene, in presence of a catalyst containing a zeolite, in conditions of transalkylation to obtain a mixture containing cumene and ethylbenzene;
(e) feeding the mixture resulting from stage (d) back to stage (c);
(f) possibly feeding the fraction (1) resulting from stage (c) back to stage (a) and/or to stage (b) and/or to stage (d).

When operating according to this particular embodiment:
- the first fraction coming from the separation section of stage (c) contains non-converted benzene coming from both the alkylation stages and the transalkylation stage,
- the second fraction coming from the separation section contains ethylbenzene coming from both the alkylation stages and the transalkylation stage,
- the third fraction coming from the separation section contains cumene coming from both the alkylation stages and the transalkylation stage,
- the fourth fraction contains polyalkylbenzenes coming from both the alkylation stages and the transalkylation stage.

The cumene obtained according to the integrated process, object of the present invention, according to any of the embodiments previously described, can be used for the production of phenol by oxidation to cumene hydroperoxide and subsequent rearrangement of the hydroperoxide to phenol and acetone.

A further object of the present invention relates in particular to a process for the production of phenol which comprises the following stages:
(a) contacting benzene and ethylene, in presence of a catalyst containing one or more zeolites;
(b) contacting the mixture resulting from the previous stage with propylene, and possibly with benzene, in presence of a catalyst containing one or more zeolites;
(c) subjecting the mixture resulting from stage (b) to separation in order to isolate at least the following fractions:
   (1) one fraction containing benzene
   (2) one fraction containing ethylbenzene,
   (3) one fraction containing cumene,
   (4) one fraction containing polyalkylbenzenes;
(d) possibly contacting the fraction (4) containing the polyalkylbenzenes with benzene, in presence of a catalyst containing a zeolite, in conditions of transalkylation to obtain a mixture containing cumene and ethylbenzene;
(e) possibly feeding the mixture resulting from stage (d) back to stage (c);
(f) possibly feeding the fraction (1) resulting from stage (c) back to stage (a) and/or to stage (b) and/or to stage (d);
(g) subjecting the fraction (3) containing cumene to oxidation to obtain cumene hydroperoxide;
(h) rearranging the cumene hydroperoxide to phenol and acetone.

A preferred aspect is to use ethylene as olefin in stage (a) and propylene as olefin in stage (b).
The various oxidation passages of cumene to cumene hydroperoxide, rearrangement of the hydroperoxide to give phenol and acetone and purification of phenol are well-known in literature, as described for example in US 5,160,497 and US 5,017,729.

The oxidation stage (g) of cumene to cumene hydroperoxide can be effected, for example, with molecular oxygen at a temperature ranging from 60 to 150°C and a pressure ranging from 1 to 10 kg-f/cm2. It is preferable to operate in the presence of an initiator and an alkaline compound for the pH control.

Stage (h) for the transformation of cumene hydroperoxide to phenol and acetone is effected in the presence of an acid, for example a strong acid such as sulphuric acid, or for example an exchanger resin or a silico-alumina. At the end, the reaction mixture is subjected to concentration to recover the acetone.

According to a preferred aspect, in the separation stage, a fraction containing benzene is isolated, to be fed back to stage (a) and/or to stage (b) and/or to stage (d), together with a fraction of polyalkylbenzenes, to be used in a separate transalkylation stage with benzene to recover further cumene and further ethylbenzene. Said transalkylation reaction is effected as described above; the resulting mixture can be subjected to separation by re-feeding to the separation stage (c), as previously described.

The ethylbenzene obtained according to the integrated process, object of the present invention, according to any of the embodiments previously described, can be used for the production of styrene by means of a dehydrogenation reaction.

A further object of the present invention relates, in particular, to a process for the production of styrene which comprises the following stages:
(a) contacting benzene and ethylene, in presence of a catalyst containing one or more zeolites;
(b) contacting the mixture resulting from the previous stage with propylene, and possibly with benzene, in presence of a catalyst containing one or more zeolites;
(c) subjecting the mixture resulting from stage (b) to separation in order to isolate at least the following fractions:
   (1) one fraction containing benzene
   (2) one fraction containing ethylbenzene,
   (3) one fraction containing cumene,
   (4) one fraction containing polyalkylbenzenes;
(d) possibly contacting the fraction (4) containing the polyalkylbenzenes with benzene, in presence of a catalyst containing a zeolite, in conditions of transalkylation to obtain a mixture containing cumene and ethylbenzene;
(e) possibly feeding the mixture resulting from stage (d) back to stage (c);
(f) possibly feeding the fraction (1) resulting from stage (c) back to stage (a) and/or to stage (b) and/or to stage (d).
(g1) subjecting the fraction containing ethylbenzene to dehydrogenation to obtain styrene.

A preferred aspect is to use ethylene as olefin in stage (a), and propylene as olefin in stage (b). The stage (g1) is well-known in literature, as described for example in US 7,393,986.

According to a preferred aspect, in the separation stage, a fraction containing benzene is isolated, to be fed back to stage (a) and/or to stage (b) and/or to stage (d), together with a fraction of polyalkylbenzenes, to be used in a separate transalkylation stage with benzene to recover further cumene and further ethylbenzene. Said transalkylation reaction is effected as described above; the resulting mixture can be subjected to separation by re-feeding to stage (c), as previously described.

As already indicated above, in all the process schemes previously described, or in the schemes obtained therefrom, stage (b) can be effected without any addition of further benzene, in addition to that already contained in the mixture resulting from stage (a), or by adding further benzene. In this latter case, fresh benzene can be used or fraction (1) containing benzene deriving from stage (c) or both: a preferred aspect is to use only fraction (1) containing benzene deriving from stage (c).

The following examples have the purpose of illustrating the invention without limiting its objectives.

### Example 1

### Stage (a) - Alkylation of benzene with ethylene

In stage (a), the catalyst based on beta zeolite prepared as described in Example 4 of EP 847 802, is used as alkylation catalyst, adopting the beta zeolite prepared as described in examples 1 and 3 of EP 847 802 and alumina in the form of bohemite. The reactor used for the catalytic test is of the Berty type, consisting of a reaction chamber having a capacity of 250 cc inside which there is a 50 cc basket in which the catalyst described above is charged. The head of the reactor is positioned in the upper part of the reaction chamber, which supports a rotor which is rotated by means of a magnetic coupling. The reactor is equipped with a temperature and pressure regulation system. Before entering the reactor, the benzene being fed is passed through an alumina column in order to reduce the quantity of water contained therein to below 50 ppm and is then fed in continuous to the reactor. The ethylene is fed by means of a mass flowmeter and mixed with benzene before entering the reactor. The conditions under which the test is carried out are the following: reaction temperature equal to 220°C, reaction pressure equal to 37 bar, space velocity expressed as LHSV equal to 3 hours⁻¹, benzene/ethylene molar ratio equal to 5. The effluent from the reactor is collected in a tank and analyzed by means of gas chromatography using an HP 5890 Series 2 instrument equipped with a capillary column with a Carbovax 20M stationary phase and detector of the flame ionization type (FID).

Under the above reaction conditions, a quantitative conversion of ethylene is obtained, with a selectivity to ethylbenzene of 80%, a selectivity of ethylene to useful aromatic compounds (intended as the sum of the desired product ethylbenzene and polyethylbenzenes recoverable by transalkylation) of 99.9% and the formation of 2.85 kg of heavy products per ton of ethylbenzene produced, wherein the heavy products contain diphenylethanes and alkyl diphenylethanes.

### Stage (b) - alkylation with propylene of the product coming from the previous stage (a)

The catalyst used is the same catalyst as the previous stage (a). The test is effected in the same reactor described in stage (a) to which the raw alkylated liquid deriving from the alkylation of benzene with ethylene is fed, after treatment on an alumina column. The propylene is fed in the liquid state by means of an isocratic pump and mixed with the alkylated liquid before entering the reactor.

The conditions under which the test is carried out are the following:
reaction temperature equal to 140°C, reaction pressure equal to 37 bar, space velocity expressed as LHSV equal to 3 hours⁻¹, benzene/propylene molar ratio equal to 2.7.

The effluent from the reactor is collected in a tank and analyzed by means of gas chromatography according to the procedure described in stage (a).

Under the above reaction conditions, a quantitative conversion of propylene is obtained, with a selectivity to cumene of 65%, a selectivity of propylene to useful aromatic compounds (intended as the sum of cumene and polyalkylbenzenes recoverable by transalkylation) of 99.9% and the formation of 2.96 kg of heavy products per ton of mono-alkylated product (ethylbenzene + cumene), wherein the heavy products contain diphenylethanes, alkyl diphenylethanes, diphenylpropanes and alkyl diphenylpropanes.

As can be observed, the additional alkylation with propylene of the alkylated liquid product coming from stage (a) does not lead to a significant deterioration in the formation of heavy products, wherein said heavy products represent the loss of material of the whole process.

## Claims

1. Integrated process for preparing cumene and ethylbenzene through alkylation of benzene comprising the following stages:
(a) contacting benzene and ethylene in presence of a catalyst containing one or more zeolites,
(b) contacting the mixture resulting from the previous stage with propylene, and possibly with benzene, in presence of a catalyst containing one or more zeolites,
(c) subjecting the mixture resulting from stage (b) to separation in order to isolate at least one fraction containing ethylbenzene and a fraction containing cumene.

2. Integrated process according to claim 1 comprising the following stages:
(a) contacting benzene and ethylene in presence of a catalyst containing one or more zeolites,
(b) contacting the mixture resulting from the previous stage with propylene, and possibly with benzene, in presence of a catalyst containing one or more zeolites,
(c) subjecting the mixture resulting from stage (b) to separation in order to isolate at least: (1) one fraction containing benzene, (2) one fraction containing ethylbenzene, (3) one fraction containing cumene, (4) one fraction containing polyalkylbenzenes.

3. Process according to claim 2 wherein the fraction (4) containing the polyalkylbenzenes, obtained in stage (c), is recycled to stage (a) and/or (b).

4. Process according to claim 2 additionally comprising a stage (d) wherein the fraction (4) containing the polyalkylbenzenes, obtained in stage (c), is reacted with benzene, in presence of a catalyst containing a zeolite, in conditions of transalkylation, to obtain cumene and ethylbenzene.

5. Process according to claim 2, 3 or 4 wherein the fraction (1) containing benzene is fed back to stage (a) and/or (b), and/or (d) if present.

6. Process according to one or more of the preceding claims wherein fresh benzene is fed to stage (b).

7. Process according to claim 1 or 2 wherein the zeolites used in stage (a) and (b) are the same.

8. Process according to claim 1 or 2 wherein the zeolites used in stage (a) and (b) are selected from zeolite beta, zeolite Y, MCM-22, zeolite ZSM-12 and mordenite.

9. Process according to one or more of claims 6 - 8 wherein zeolite beta is used both in stage (a) and in stage (b).

10. Process according to one or more of the preceding claims wherein the zeolites used in stage (a) and (b) are in acid form.

11. Process according to claim 1 or 2 wherein the reactions are performed continuously and in liquid phase both in stage (a) and in stage (b).

12. Process according to claim 1 or 2 wherein the reactions of stage (a) and (b) are performed at a reaction temperature comprised between 100 and 300°C, the pressure is equivalent or greater than the atmospheric pressure and the space velocity WHSV is comprised between 0.1 and 10 hours⁻¹.

13. Process according to claim 12 wherein the temperature is comprised between 120 and 250 °C and the pressure is comprised between 10 and 50 bars.

14. Process according to claim 4 wherein in stage (d) the catalyst contains zeolite Y.

15. Process according to one or more of the preceding claims comprising the following stages:
(a) contacting benzene and ethylene in presence of a catalyst containing a zeolite,
(b) contacting the mixture resulting from the previous stage with propylene, and possibly with benzene, in presence of a catalyst containing a zeolite,
(c) subjecting the mixtures resulting from stage (b) and from stage (d) to separation in order to isolate at least: (1) one fraction containing benzene, (2) one fraction containing ethylbenzene, (3) one fraction containing cumene, (4) one fraction containing polyalkylbenzenes;
(d) contacting the fraction (4) containing the polyalkylbenzenes with benzene, in presence of a catalyst containing a zeolite, in conditions of transalkylation to obtain a mixture containing cumene and ethylbenzene;
(e) feeding the mixture resulting from stage (d) back to stage (c);
(f) possibly feeding the fraction (1) resulting from stage (c) back to stage (a) and/or to stage (b) and/or to stage (d).

16. Process according to one or more of the preceding claims additionally comprising a stage wherein the cumene obtained in stage (c) is subjected to oxidation to generate cumene hydroperoxide, and a stage wherein the cumene hydroperoxide is subjected to rearrangement to generate phenol and acetone.

17. Process according to claim 16 for the production of phenol comprising the following steps:
(a) contacting benzene and ethylene, in presence of a catalyst containing a zeolite,
(b) contacting the mixture resulting from the previous stage with propylene, and possibly with benzene, in presence of a catalyst containing a zeolite,
(c) subjecting the mixture resulting from stage (b) to separation in order to isolate at least the following fractions:
(1) one fraction containing benzene
(2) one fraction containing ethylbenzene,
(3) one fraction containing cumene,
(4) one fraction containing polyalkylbenzenes,
(d) possibly contacting the fraction (4) containing the polyalkylbenzenes with benzene, in presence of a catalyst containing a zeolite, in conditions of transalkylation to obtain a mixture containing cumene and ethylbenzene,
(e) possibly feeding the mixture resulting from stage (d) back to stage (c);
(f) possibly feeding the fraction (1) resulting from stage (c) back to stage (a) and/or to stage (b) and/or to stage (d).
(g) subjecting the fraction (3) containing cumene to oxidation to obtain cumene hydroperoxide
(h) rearranging the cumene hydroperoxide to phenol and acetone.

18. Process according to one or more of the preceding claims 1 - 15 additionally comprising a stage wherein the ethylbenzene obtained in stage (c) is subjected to dehydrogenation to obtain styrene.

19. Process according to claim 18 for the production of styrene comprising the following steps:
(a) contacting benzene and ethylene, in presence of a catalyst containing a zeolite,
(b) contacting the mixture resulting from the previous stage with propylene, and possibly with benzene, in presence of a catalyst containing a zeolite,
(c) subjecting the mixture resulting from stage (b) to separation in order to isolate at least the following fractions:
(1) one fraction containing benzene
(2) one fraction containing ethylbenzene,
(3) one fraction containing cumene,
(4) one fraction containing polyalkylbenzenes,
(d) possibly contacting the fraction (4) containing the polyalkylbenzenes with benzene, in presence of a catalyst containing a zeolite, in conditions of transalkylation to obtain a mixture containing cumene and ethylbenzene,
(e) possibly feeding the mixture resulting from stage (d) back to stage (c);
(f) possibly feeding the fraction (1) resulting from stage (c) back to stage (a) and/or to stage (b) and/or to stage (d).
(g1) subjecting the fraction containing ethylbenzene to dehydrogenation to obtain styrene.

## Patentansprüche

1. Integriertes Verfahren zur Herstellung von Cumen und Ethylbenzen durch Alkylierung von Benzen, umfassend die folgenden Stufen:
(a) Kontaktierung von Benzen und Ethylen in Gegenwart eines Katalysatoren, der einen oder mehrere Zeolithe enthält,
(b) Kontaktierung der in der vorhergehenden Stufe erhaltenen Mischung mit Propylen und gegebenenfalls mit Benzen in Gegenwart eines Katalysatoren, der einen oder mehrere Zeolithe enthält,
(c) Unterwerfung der aus Stufe (b) erhaltenen Mischung einer Auftrennung, um mindestens eine Fraktion zu isolieren, die Ethylbenzen enthält, und
eine Fraktion, die Cumen enthält.

2. Integriertes Verfahren nach Anspruch 1, umfassend die folgenden Stufen:
(a) Kontaktierung von Benzen und Ethylen in Gegenwart eines Katalysatoren, der einen oder mehrere Zeolithe enthält,
(b) Kontaktierung der in der vorhergehenden Stufe erhaltenen Mischung mit Propylen und gegebenenfalls mit Benzen in Gegenwart eines Katalysatoren, der einen oder mehrere Zeolithe enthält,
(c) Unterwerfung der aus Stufe (b) erhaltenen Mischung einer Auftrennung, um mindestens zu isolieren: (1) eine Fraktion, die Benzen enthält, (2) eine Fraktion, die Ethylbenzen enthält, (3) eine Fraktion, die Cumen enthält, (4) eine Fraktion, die Polyalkylbenzene enthält.

3. Verfahren nach Anspruch 2, worin die Fraktion (4), die Polyalkylbenzene enthält, erhalten in Stufe (c), zur Stufe (a) und/oder (b) zurückgeführt wird.

4. Verfahren nach Anspruch 2, gegebenenfalls umfassend eine Stufe (d), worin die Fraktion (4), die Polyalkylbenzene enthält, erhalten in Stufe (c), mit Benzen umgesetzt wird in Gegenwart eines Katalysatoren, der einen Zeolith enthält, unter Bedingungen der Transalkylierung, um Cumen und Ethylbenzen zu erhalten.

5. Verfahren nach Anspruch 2, 3 oder 4, worin die Benzen-enthaltende Fraktion (1) der Stufe (a) und/oder (b), und/oder soweit vorhanden (d) zurückgeführt wird.

6. Verfahren nach einem oder mehreren der vorhergehenden Ansprüche, worin frisches Benzen der Stufe (b) zugespeist wird.

7. Verfahren nach Anspruch 1 oder 2, worin die in Stufe (a) und (b) verwendeten Zeolithe die gleichen sind.

8. Verfahren nach Anspruch 1 oder 2, worin die in Stufe (a) und (b) verwendeten Zeolithe ausgewählt sind aus Zeolith Beta, Zeolith Y, MCM-22, Zeolith ZSM-12 und Mordenit.

9. Verfahren nach einem oder mehreren der Ansprüche 6 - 8, worin Zeolith Beta sowohl in Stufe (a) als auch in Stufe (b) verwendet wird.

10. Verfahren nach einem oder mehreren der vorhergehenden Ansprüche, worin die in Stufe (a) und (b) verwendeten Zeolithe in Säureform vorliegen.

11. Verfahren nach Anspruch 1 oder 2, worin die Reaktionen kontinuierlich durchgeführt werden und in flüssiger Phase sowohl in Stufe (a) als auch in Stufe (b).

12. Verfahren nach Anspruch 1 oder 2, worin die Reaktionen der Stufe (a) und (b) bei einer Reaktionstemperatur in einem Bereich zwischen 100 und 300°C durchgeführt werden, der Druck ist gleichwertig oder größer als Atmosphärendruck und die Raumgeschwindigkeit WHSV liegt im einem Bereich zwischen 0,1 und 10 Stunden-¹.

13. Verfahren nach Anspruch 12, worin die Temperatur in einem Bereich zwischen 120 und 250°C und der Druck in einem Bereich zwischen 10 und 50 bar liegen.

14. Verfahren nach Anspruch 4, worin in Stufe (d) der Katalysator Zeolith Y enthält.

15. Verfahren nach einem oder mehreren der vorhergehenden Ansprüche, umfassend die folgenden Stufen:
(a) Kontaktierung von Benzen und Ethylen in Gegenwart eines Katalysatoren, der einen Zeolith enthält,
(b) Kontaktierung der in der vorhergehenden Stufe erhaltenen Mischung mit Propylen und gegebenenfalls mit Benzen in Gegenwart eines Katalysatoren, der einen Zeolith enthält,
(c) Unterwerfung der aus Stufe (b) und Stufe (d) erhaltenen Mischungen einer Auftrennung, um mindestens zu isolieren: (1) eine Fraktion, die Benzen enthält, (2) eine Fraktion, die Ethylbenzen enthält, (3) eine Fraktion, die Cumen enthält, (4) eine Fraktion, die Polyalkylbenzene enthält;
(d) Kontaktierung der Fraktion (4), die Polyalkylbenzene mit Benzen enthält, in Gegenwart eines Katalysatoren, der Zeolith enthält, unter Bedingungen der Transalkylierung, um eine Mischung zu erhalten, die Cumen und Ethylbenzen enthält;
(e) Einspeisung der in Stufe (d) erhaltenen Mischung zurück zur Stufe (c);
(f) gegebenenfalls Einspeisung der Fraktion (1), die in Stufe (c) erhalten wurde, zurück zur Stufe (a) und/oder zur Stufe (b) und /oder zur Stufe (d).

16. Verfahren nach einem oder mehreren der vorhergehenden Ansprüche, zusätzlich umfassend eine Stufe, worin das in Stufe (c) erhaltene Cumen einer Oxidierung unterworfen wird, um Cumenhydroperoxid zu erhalten, und eine Stufe, worin das Cumenhydroperoxid einer Umlagerung unterworfen wird, um Phenol und Aceton zu erzeugen.

17. Verfahren nach Anspruch 16, zur Herstellung von Phenol, umfassend die folgenden Schritte:
(a) Kontaktierung von Benzen und Ethylen in Gegenwart eines Katalysatoren, der einen Zeolith enthält,
(b) Kontaktierung der in der vorhergehenden Stufe erhaltenen Mischung mit Propylen und gegebenenfalls mit Benzen in Gegenwart eines Katalysatoren, der einen Zeolith enthält,
(c) Unterwerfung der aus Stufe (b) erhaltenen Mischung einer Auftrennung, um mindestens die folgenden Fraktionen zu isolieren:
(1) eine Fraktion, die Benzen enthält,
(2) eine Fraktion, die Ethylbenzen enthält,
(3) eine Fraktion, die Cumen enthält,
(4) eine Fraktion, die Polyalkylbenzene enthält,
(d) gegebenenfalls Kontaktierung der Fraktion (4), die Polyalkylbenzene mit Benzen enthält, in Gegenwart eines Katalysatoren, der Zeolith enthält, unter Bedingungen der Transalkylierung, um eine Mischung zu erhalten, die Cumen und Ethylbenzen enthält;
(e) gegebenenfalls Einspeisung der in Stufe (d) erhaltenen Mischung zurück zur Stufe (c);
(f) gegebenenfalls Einspeisung der Fraktion (1), die in Stufe (c) erhalten wurde, zurück zur Stufe (a) und/oder zur Stufe (b) und /oder zur Stufe (d);
(g) Unterwerfung der Fraktion (3), die Cumen enthält, einer Oxidation, um Cumenhydroperoxid zu erhalten;
(h) Umlagerung des Cumenhydroperoxids in Phenol und Aceton.

18. Verfahren nach einem oder mehreren der vorhergehenden Ansprüche 1 - 15, zusätzlich umfassend eine Stufe, in der das in Stufe (c) erhaltene Ethylbenzen einer Dehydrogenierung unterworfen wird, um Styren zu erhalten.

19. Verfahren nach Anspruch 18 zur Herstellung von Styren, umfassend die folgenden Schritte:
(a) Kontaktierung von Benzen und Ethylen in Gegenwart eines Katalysatoren, der einen Zeolith enthält,
(b) Kontaktierung der in der vorhergehenden Stufe erhaltenen Mischung mit Propylen und gegebenenfalls mit Benzen in Gegenwart eines Katalysatoren, der einen Zeolith enthält,
(c) Unterwerfung der aus Stufe (b) erhaltenen Mischung einer Auftrennung, um mindestens die folgenden Fraktionen zu isolieren:
(1) eine Fraktion, die Benzen enthält,
(2) eine Fraktion, die Ethylbenzen enthält,
(3) eine Fraktion, die Cumen enthält,
(4) eine Fraktion, die Polyalkylbenzene enthält,
(d) gegebenenfalls Kontaktierung der Fraktion (4), die Polyalkylbenzene enthält, mit Benzen in Gegenwart eines Katalysatoren, der Zeolith enthält, unter Bedingungen der Transalkylierung, um eine Mischung zu erhalten, die Cumen und Ethylbenzen enthält;
(e) gegebenenfalls Einspeisung der in Stufe (d) erhaltenen Mischung zurück zur Stufe (c);
(f) gegebenenfalls Einspeisung der Fraktion (1), die in Stufe (c) erhalten wurde, zurück zur Stufe (a) und/oder zur Stufe (b) und /oder zur Stufe (d);
(g1) Unterwerfung der Fraktion, die Ethylbenzen enthält, einer Dehydrogenierung, um Styren zu erhalten.

## Revendications

1. Procédé intégré de préparation de cumène et d'éthyl-benzène par alkylation du benzène, comportant les étapes suivantes :
a) mettre en contact du benzène et de l'éthylène, en présence d'un catalyseur comprenant une ou plusieurs zéolithe(s) ;
b) mettre le mélange résultant de l'étape précédente en contact avec du propylène, et en option, avec du benzène, en présence d'un catalyseur comprenant une ou plusieurs zéolithe(s) ;
c) soumettre le mélange résultant de l'étape (b) à une opération de séparation, afin d'isoler au moins une fraction contenant de l'éthyl-benzène et une fraction contenant du cumène.

2. Procédé intégré conforme à la revendication 1, comportant les étapes suivantes :
a) mettre en contact du benzène et de l'éthylène, en présence d'un catalyseur comprenant une ou plusieurs zéolithe(s) ;
b) mettre le mélange résultant de l'étape précédente en contact avec du propylène, et en option, avec du benzène, en présence d'un catalyseur comprenant une ou plusieurs zéolithe(s) ;
c) soumettre le mélange résultant de l'étape (b) à une opération de séparation, afin d'isoler au moins
1) une fraction contenant du benzène,
2) une fraction contenant de l'éthyl-benzène,
3) une fraction contenant du cumène,
4) et une fraction contenant des polyalkyl-benzènes.

3. Procédé conforme à la revendication 2, dans lequel la fraction (4) contenant des polyalkyl-benzènes, obtenue à l'issue de l'étape (c), est recyclée dans l'étape ou les étapes (a) et/ou (b).

4. Procédé conforme à la revendication 2, comportant en plus une étape (d) dans laquelle on fait réagir la fraction (4) contenant des polyalkyl-benzènes, obtenue à l'issue de l'étape (c), avec du benzène, en présence d'un catalyseur contenant une zéolithe et dans des conditions de trans-alkylation, pour obtenir du cumène et de l'éthyl-benzène.

5. Procédé conforme à la revendication 2, 3 ou 4, dans lequel la fraction (1) contenant du benzène est renvoyée en alimentation à l'étape ou aux étapes (a) et/ou (b), et/ou (d) si celle-ci est réalisée.

6. Procédé conforme à l'une ou plusieurs des revendications précédentes, dans lequel du benzène neuf est envoyé en alimentation à l'étape (b).

7. Procédé conforme à la revendication 1 ou 2, dans lequel les zéolithes utilisées dans les étapes (a) et (b) sont les mêmes.

8. Procédé conforme à la revendication 1 ou 2, dans lequel les zéolithes utilisées dans les étapes (a) et (b) sont choisies parmi les suivantes : zéolithe bêta, zéolithe Y, MCM-22, zéolithe ZSM-12, et mordénite.

9. Procédé conforme à l'une ou plusieurs des revendications 6 à 8, dans lequel une zéolithe bêta est utilisée dans les deux étapes (a) et (b).

10. Procédé conforme à l'une ou plusieurs des revendications précédentes, dans lequel les zéolithes utilisées dans les étapes (a) et (b) sont sous leur forme acide.

11. Procédé conforme à la revendication 1 ou 2, dans lequel les réactions, dans les deux étapes (a) et (b), sont réalisées en mode continu et en phase liquide.

12. Procédé conforme à la revendication 1 ou 2, dans lequel les réactions des étapes (a) et (b) sont réalisées à une température de réaction située entre 100 et 300 °C, sous une pression équivalente ou supérieure à la pression atmosphérique, et avec une vitesse spatiale horaire en poids (VSHP) valant de 0,1 à 10 h⁻¹.

13. Procédé conforme à la revendication 12, dans lequel la température est située entre 120 et 250 °C et la pression vaut entre 10 et 50 bars.

14. Procédé conforme à la revendication 4, dans lequel, dans l'étape (d), le catalyseur contient une zéolithe Y.

15. Procédé conforme à l'une ou plusieurs des revendications précédentes, comportant les étapes suivantes :
a) mettre en contact du benzène et de l'éthylène, en présence d'un catalyseur comprenant une zéolithe ;
b) mettre le mélange résultant de l'étape précédente en contact avec du propylène, et en option, avec du benzène, en présence d'un catalyseur comprenant une zéolithe ;
c) soumettre les mélanges résultant de l'étape (b) et de l'étape (d) à une opération de séparation, afin d'isoler au moins
1) une fraction contenant du benzène,
2) une fraction contenant de l'éthyl-benzène,
3) une fraction contenant du cumène,
4) et une fraction contenant des polyalkyl-benzènes ;
d) mettre la fraction (4) contenant des polyalkyl-benzènes en contact avec du benzène, en présence d'un catalyseur contenant une zéolithe et dans des conditions de trans-alkylation, pour obtenir un mélange contenant du cumène et de l'éthyl-benzène ;
e) renvoyer le mélange résultant de l'étape (d) en alimentation à l'étape (c) ;
f) en option, renvoyer la fraction (1) résultant de l'étape (c) en alimentation à l'étape (a) et/ou à l'étape (b) et/ou l'envoyer à l'étape (d).

16. Procédé conforme à l'une ou plusieurs des revendications précédentes, qui comporte en plus une étape dans laquelle on soumet à une oxydation le cumène obtenu à l'issue de l'étape (c), pour produire de l'hydroperoxyde de cumène, ainsi qu'une étape dans laquelle on soumet cet hydroperoxyde de cumène à une réaction de réarrangement, pour produire du phénol et de l'acétone.

17. Procédé de production de phénol, conforme à la revendication 16, comportant les étapes suivantes :
a) mettre en contact du benzène et de l'éthylène, en présence d'un catalyseur comprenant une zéolithe ;
b) mettre le mélange résultant de l'étape précédente en contact avec du propylène, et en option, avec du benzène, en présence d'un catalyseur comprenant une zéolithe ;
c) soumettre le mélange résultant de l'étape (b) à une opération de séparation, afin d'isoler au moins les fractions suivantes :
1) une fraction contenant du benzène,
2) une fraction contenant de l'éthyl-benzène,
3) une fraction contenant du cumène,
4) et une fraction contenant des polyalkyl-benzènes ;
d) en option, mettre la fraction (4) contenant des polyalkyl-benzènes en contact avec du benzène, en présence d'un catalyseur contenant une zéolithe et dans des conditions de trans-alkylation, pour obtenir un mélange contenant du cumène et de l'éthyl-benzène ;
e) en option, renvoyer le mélange résultant de l'étape (d) en alimentation à l'étape (c) ;
f) en option, renvoyer la fraction (1) résultant de l'étape (c) en alimentation à l'étape (a) et/ou à l'étape (b) et/ou l'envoyer à l'étape (d) ;
g) soumettre à une oxydation la fraction (3) contenant du cumène, pour obtenir de l'hydroperoxyde de cumène ;
h) opérer un réarrangement de cet hydroperoxyde de cumène en phénol et acétone.

18. Procédé conforme à l'une ou plusieurs des revendications 1 à 15, qui comporte en plus une étape dans laquelle on soumet l'éthyl-benzène obtenu à l'issue de l'étape (c) à une déshydrogénation, pour obtenir du styrène.

19. Procédé de production de styrène, conforme à la revendication 18, comportant les étapes suivantes :
a) mettre en contact du benzène et de l'éthylène, en présence d'un catalyseur comprenant une zéolithe ;
b) mettre le mélange résultant de l'étape précédente en contact avec du propylène, et en option, avec du benzène, en présence d'un catalyseur comprenant une zéolithe ;
c) soumettre le mélange résultant de l'étape (b) à une opération de séparation, afin d'isoler au moins les fractions suivantes :
1) une fraction contenant du benzène,
2) une fraction contenant de l'éthyl-benzène,
3) une fraction contenant du cumène,
4) et une fraction contenant des polyalkyl-benzènes ;
d) en option, mettre la fraction (4) contenant des polyalkyl-benzènes en contact avec du benzène, en présence d'un catalyseur contenant une zéolithe et dans des conditions de trans-alkylation, pour obtenir un mélange contenant du cumène et de l'éthyl-benzène ;
e) en option, renvoyer le mélange résultant de l'étape (d) en alimentation à l'étape (c) ;
f) en option, renvoyer la fraction (1) résultant de l'étape (c) en alimentation à l'étape (a) et/ou à l'étape (b) et/ou l'envoyer à l'étape (d) ;
gl) soumettre à une déshydrogénation la fraction contenant de l'éthyl-benzène, pour obtenir du styrène.
